Europäisches Patentamt

European Patent Office

Office européen des brevets

(11) Publication number: **0 329 203 B1**

## EUROPEAN PATENT SPECIFICATION

(45) Date of publication of patent specification: **01.09.93**

(51) Int. Cl.⁵: **C12N 15/81**, C12N 15/51

(21) Application number: **89106868.6**

(22) Date of filing: **06.01.84**

(60) Publication number of the earlier application in accordance with Art.76 EPC: **0 120 551**

Divisional application 91114001.0 filed on 06/01/84.

(54) **Yeast expression systems with vectors having PyK promoters, and synthesis of foreign protein.**

(30) Priority: **22.02.83 US 468589**

(43) Date of publication of application:
**23.08.89 Bulletin 89/34**

(45) Publication of the grant of the patent:
**01.09.93 Bulletin 93/35**

(84) Designated Contracting States:
**AT BE CH DE FR GB IT LI LU NL SE**

(56) References cited:
**EP-A- 0 072 318**

**BIOCHEMICAL AND BIOPHYSICAL RESEARCH COMMUNICATIONS, vol. 108, no. 3, 15th October 1982, pages 1107-1112, Academic Press, Inc.; G. KAWASAKI et al.: "Cloning of yeast glycolysis genes by complementation"**

**PROCEEDINGS OF THE NATIONAL ACADEMY OF SCIENCES OF THE USA, vol. 80, no. 1, January 1983, pages 1-5, Washington, US; A. MIYANOHARA et al.: "Expression of hepatitis B surface antigen gene in yeast"**

(73) Proprietor: **CHIRON CORPORATION**
**4560 Horton Street**
**Emeryville, California 94608(US)**

(72) Inventor: **Burke,Rae Lyn**
**1624 8th Avenue**
**San Francisco,California(US)**
Inventor: **Tekamp/Olson,Patricia**
**1426 43rd Avenue**
**San Francisco,California(US)**
Inventor: **Rosenberg,Steven**
**3861 Fruitvale Avenue**
**Oakland,California(US)**
Inventor: **Valenzuela,Pablo D.T.**
**455 Upper Terrace 3**
**San Francisco,California(US)**

(74) Representative: **Glawe, Delfs, Moll & Partner**
**Patentanwälte, Postfach 26 01 62**
**D-80058 München (DE)**

COLD SPRING HARBOR CONFERENCE ON MODERN APPROACHES TO VACCINES, MOLECULAR AND CHEMICAL BASIS OF VIRUS VIRULENCE AND IMMUNOGENICITY, September 1983, Conference Proceedings, pages 209-213; P. VALENZUELA et al.: "Hepatitis-B vaccine: characterization of hepatitis-B antigen particles produced in yeast"

## Description

### BACKGROUND OF THE INVENTION

For maximal expression of foreign genes in microbial systems it is usually advantageous to employ homologous regulatory elements within the expression vector. Efficiency of expression (product formation) is believed to be a function of and proportional to the strength of the promoter employed. In addition, regulation of gene expression by nutritional factors under the control of the experimenter offers a further useful manipulatory tool. The glycolytic enzyme genes of yeast, e.g., those coding for glyceraldehyde-3-phosphate dehydrogenase (GAPDH) and pyruvate kinase (PyK), possess the above useful properties, i.e., high levels of expression (and thus by inference very efficient promoters) and susceptibility to regulation by components of the growth medium. For example, GAPDH can comprise as much as 5 % of the dry weight of commercial baker's yeast (Krebs, E.G., J. Biol. Chem. (1953) 200:471). Furthermore, these enzymes are also high inducible. For example, when yeast cultures are shifted from growth on acetate to glucose, the activity of GAPDH increased up to 200-fold in proportion to the concentration of the sugar in the medium (Maitra, P.K. and Lobo, Z., J. Biol. Chem. (1971) 246:475). These results suggest that the transcriptional machinery of these genes is highly regulated, perhaps by the participation of DNA sequences present in the 5′ non-coding flanking region of the genes.

This invention relates to the isolation, structure and the successful use in yeast expression plasmids of DNA fragments corresponding to the 5′ non-coding regions of the regulatable yeast genes GAPDH and PyK. These fragments which contain DNA sequences with strong transcription-promoting activity are called "promoters". They are ideal components of DNA vectors for commercial production of large quantities of protein coded by foreign genes under their transcriptional control.

In addition, this invention encompasses yeast expression plasmids further comprising an appropriate terminator to a form a "cassette" of promoter-foreign gene-terminator. The presence of the terminator increases expression of the foreign DNA.

An early attempt to express foreign DNA in yeast failed (Beggs, J.D. et al., Nature (1980) 283:285). In this report, the hemoglobin DNA (inserted with its own promoter) was transcribed but the RNA was not spliced. A variety of explanations for this result are possible, e.g., an incorrect location for the initiation of transcription and/or the poor ability of yeast cells to carry out splicing of intervening sequences (introns).

Three GAPDH genes of yeast have been cloned (Holland, M.J. et al., Basic Life Science (1981) 19:291), but their promoters have not been used for constructing expression systems in yeast by recombinant DNA methods. The PyK gene has also been cloned, but by genetic complementation only (no structural studies performed) (Kawasaki, G. and Fraenel, D.G., Biochem. Biophys. Res. Comm. (1982) 108:1107). Other yeast promoters, e.g., that of alcohol dehydrogenase I (Valenzuela, P. et al., Nature (1982) 298:347 and Hitzeman, R.A. et al., Nature (1981) 293:717) and phosphoglycerate kinase (Tuite, M.F. et al., EMBO J. (1982) 1:603 and Hitzeman, R.A. et al., Science (1983) 219:620) have been linked to foreign genes to produce yeast expression but no terminators were used. The present invention provides new promoters for yeast expression systems and combines the advantages of highly expressive promoters with the enhanced expression found with appropriately ligated terminators.

A published European Patent Application (072 318) disclosed the construction of a yeast expression vector which, upon induction, expressed hepatitis B virus surface antigen (HBsAg) S-protein under control of the yeast alcohol dehydrogenase I (ADHI) promoter.

### BRIEF DESCRIPTION OF THE INVENTION

This invention relates to a yeast expression vector comprising a segment of foreign DNA, e.g., that coding for hepatitis B virus (HBV) surface antigen (HBsAG), under transcriptional control of a yeast PyK promoter. Terminators may also be appropriately attached. The expression vector typically has a yeast replication origin and is capable of replicating in either type of cell. The expression vector, when used to transform yeast cells, will yield substantial amounts of the protein coded by the segment of foreign DNA.

### BRIEF DESCRIPTION OF THE DRAWINGS

Fig. 1: Isolation and tailoring of a GAPDH promoter fragment.
Fig. 2: DNA sequence of the GAPDH promoter fragment.
Fig. 3: Construction of a yeast expression plasmid containing the GAPDH promoter.
Fig. 4: Nucleotide sequence of the pyruvate kinase (PyK) gene.

Fig. 5: Construction of a yeast expression plasmid containing the PyK promoter region.

DETAILED DESCRIPTION OF THE INVENTION

In principle, yeast expression plasmids have particular advantages, including the following. Yeast can be grown in large-scale culture for commercial production by processes well-known in the art. In contrast, bacteria in large-scale culture are subject to - the frequent problem of "phage-out". Yeast also appears to have much the same ability as mammalian cells to add carbohydrate groups to newly synthesized proteins, a capacity that bacteria do not have. Now that cDNA sequences are readily obtainable, the problem of expressing genes having introns is easily avoided.

The vectors of the present invention encompass promoters of unusually high efficiency. A promoter is defined herein as a DNA segment capable of functioning to initiate transcription of an adjoining DNA segment. Transcription is the synthesis of RNA (herein termed messenger RNA or mRNA), complementary to one strand of the DNA adjoining the promoter region. In eukaryotes, messenger RNA synthesis is catalyzed by an enzyme termed RNA polymerase II. The minimum essential elements of promoter function are the following: To provide a starting point for the initiation of transcription and to provide a binding site for RNA polymerase II near the start site permitting selection of the proper strand of DNA as a template for messenger RNA synthesis. In addition, a eukaryotic promoter functions to regulate the relative efficiency of transcription of coding segments under its control. An active promoter is one which elicits synthesis of relatively large amounts of mRNA complementary to a strand of the adjacent DNA coding segment.

The structural correlates of promoter function have not been clearly established. A promoter segment usually can be identified in nature as a region lying adjacent to the 5′ end of a given structural gene. (references to the 5′ and 3′ ends of a gene will be understood to indicate the corresponding respective ends of mRNA transcribed therefrom, and these, in turn, will be understood to correlate with the $NH_2$- and -COOH termini of the encoded protein, respectively.) Comparisons of the nucleotide sequences of promoters for various genes from various species have revealed only a few short regions of nucleotide sequence similarity in common among them. Most notable of these is the "TATA Box," a segment of about 5 to 10 nucleotides located generally about 70 to 230 nucleotides upstream from the site of transcription initiation, having a sequence generally resembling TATAA. For review of structural comparisons see Breathnach, R. and Chambon, P., Ann. Rev. of Biochem. (1981) 50:349. The TATA Box is believed to function in initiation of transcription.

The foreign gene will be free or substantially free of codons from the normal structural gene associated with the promoter. Usually, the foreign gene will be joined to a non-coding 3′-end of the regulatory region encompassing the promoter, so as to be free of the amino acids at the N-terminus of endogenous gene naturally associated with the regulatory region. That is, fewer than about 3 codons (9 nucleotides) will be retained with the regulatory region when joined to the foreign gene.

The presence of the terminator sequence at the 3′ end of the coding segment enhances expression. The effect is generally similar to the addition of rho factor to prokaryotic transcription systems, wherein the rate of the release of RNA polymerase is enhanced to produce an increase in the rate of reinitiation of transcription. It will be understood that, while the terminator sequences are not required for detectable expression of foreign DNA segments, it is preferable to appropriately link them to enhance expression. The terminator region may be naturally associated with the same or different structural gene as the promoter region.

The most appropriate DNA vector for the PyK construction of this invention is a shuttle vector. These vectors can "shuttle" between a bacterial strain, such as E. coli, and yeast, since they have a bacterial origin of replication and a yeast origin of replication, see, e.g., Ammerer, G. et al., Recombinant DNA, Proc. Third Cleveland Symposium Macromolecules (Walton, A.G., ed.), p. 185, Elsevier, Amsterdam (1981). A typical bacterial origin of replication is derived from e.g., pBR322. The most useful yeast origin of replication is found in the extrachromosomal genetic element known as the 2 micron circle. In laboratory strains the 2 micron plasmid DNA is found in approximately 50 copies per cell and is stably maintained. For a review, see, for example, Curr. Topics Micro. Imm. (1982) 96:119. This yeast plasmid has also be sequenced (Hartley, J.L. et al., Nature (1980) 286:860).

Representative samples of the plasmids and host cells used in the constructions of this invention have been placed on deposit with the Americal Type Culture Collection, 12301 Parklawn Drive, Rockville, Maryland. Plasmid pPyK 9.1.1 and yeast cell transformants 2150-2-3/pHBS-56 GAP347/33 and 2150-2-3/pHBS56PyK were placed on deposit on February 18, 1983 and have received ATCC Accession numbers 40061, 20665 and 20666, respectively.

In the Examples that follow, many of the techniques, reactions and separation procedures are already well-known in the art. All enzymes, unless otherwise stated, are available from one or more commercial sources, such as New England Biolabs, Beverly, Massachusetts; Collaborative Research, Waltham, Massachusetts; Miles Laboratories, Elkhart, Indiana; Boehringer Biochemicals, Inc., Indianapolis, Indiana and Bethesda Research Laboratories, Rockville, Maryland. Buffers and reaction conditions for restriction enzyme digestion were used according to recommendations supplied by the manufacturer for each enzyme, unless otherwise indicated. Standard methodology for other enzyme reactions, gel electrophoresis separations and E. coli transformation may be found in Methods in Enzymology, (1979) 68. Transformation of yeast protoplasts can be carried out essentially as described by Beggs, Nature (1978) 275:104. E. coli strains useful for transformation include X1776; K12 strain 294 (ATCC No. 31446); RR1 and HB101. Yeast strains XV610-8c having the genotype (a ade2 ade6 leu2 lys1 trp1 can1) and GM-3C-2, genotype: (Leu2 Trp1 His4 CYC1-1CYP3-1) (Faye, G. et al., Proc. Natl. Acad. Sci. (1981) 78:2258) can be typically used for yeast transformations. It would be understood however, that virtually any strain of yeast is useful for transformation. Bacteria can be grown and selected according to procedures described by Miller, J.H., Experiments in Molecular Genetics, Cold Spring Harbor Laboratory, Cold Spring Harbor, New York (1972). Yeast can be grown on the following media: YEPD containing 1% (w/v) yeast extract, 2% (w/v) peptone and (w/v) glucose; and, in the case of plating medium, 3% (w/v) agar. YNB plus CAA contains 6.7 grams of yeast nitrogen base (Difco Laboratories, Minneapolis, Minnesota), 10mg of adenine, 10mg of uracil, 5g casamino acids (CCA) (Difco), 20g glucose; and, in the case of plating media, 30g agar per liter. Selection of tryptophan prototrophy can be made on plates containing 6.7g yeast nitrogen base (lacking amino acids), supplemented for all growth requirements of the strain to be transformed except tryptophan.

REFERENCE EXAMPLE 1

Cloning of the yeast glyceraldehyde-3-phosphate dehydrogenase (GAPDH) gene.

A complementary DNA (cDNA) containing the yeast GAPDH coding sequences was prepared in the following manner:

PolyA + RNA was isolated from yeast strain A364A. Double-stranded cDNA was synthesized using AMV reverse transcriptase and E. coli DNA polymerase I. Poly-dC-tails were added to the double-stranded cDNA molecule using deoxynucleotide terminal transferase. Poly-dC-tailed cDNA was annealed to poly-dG-tailed pBR322 and used to transform E. coli HB101. One thousand transformants were screened by colony hybridization to labeled PolyA + RNA, and a subset further examined by restriction endonuclease mapping, and DNA sequencing. Three clones containing GAPDH sequences were isolated from the pool. One clone (pcGAP-9) contained an insert of about 1200 base pairs (bp) and was used for further work.

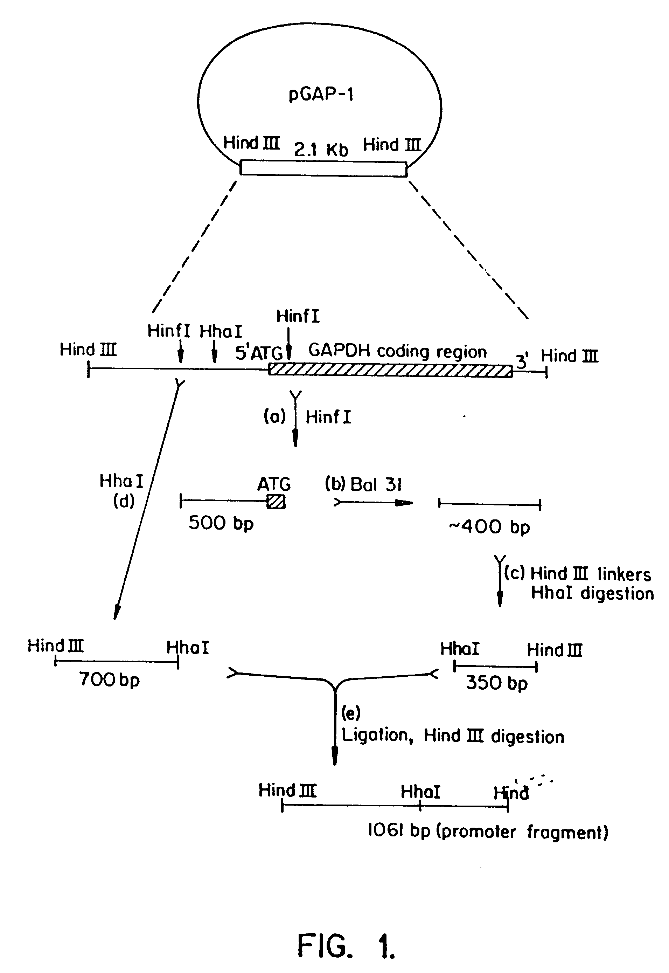

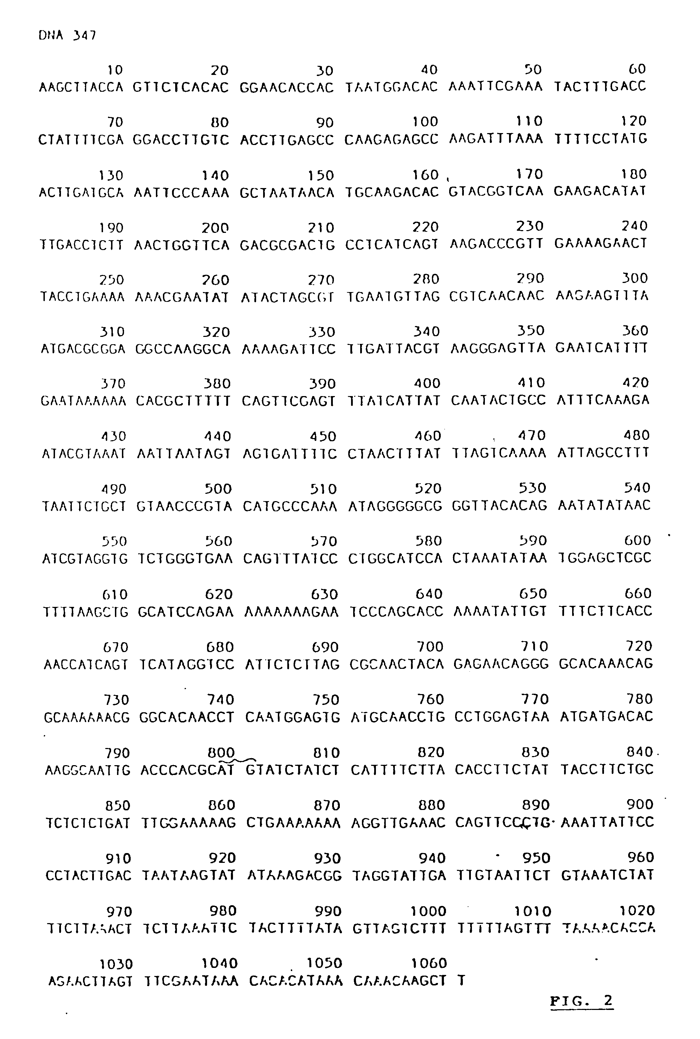

A yeast gene library was prepared by inserting fragments obtained after partial digestion of total yeast DNA with restriction endonuclease Sau3A into lambda phase Charon 28, according to Blattner, F.R. et al., Science (1977) 196:161-169. Several fragments containing yeast GAPDH coding sequences were isolated by screening the phage library with labeled DNA from pcGAP-9. The yeast GAPDH gene of one of these clones was subcloned in pBR322 as a 2.1kb HindIII fragment (pGAP-1, see Fig. 1) or as a 3.5kb BamHI fragment (pGAP-2). The GAPDH promoting-acting fragments were isolated from these clones. The HindIII-HhaI fragment of about 800bp was ligated to the HhaI-HindIII fragment of about 350bp. The resulting 1061bp HinIII fragment was isolated by gel electrophoresis and cloned in pBR322, (pGAP-347), and the sequence determined (see Fig. 2).

REFERENCE EXAMPLE 2

Construction of yeast vectors containing the GAPDH promoter, active in the expression of HBsAg.

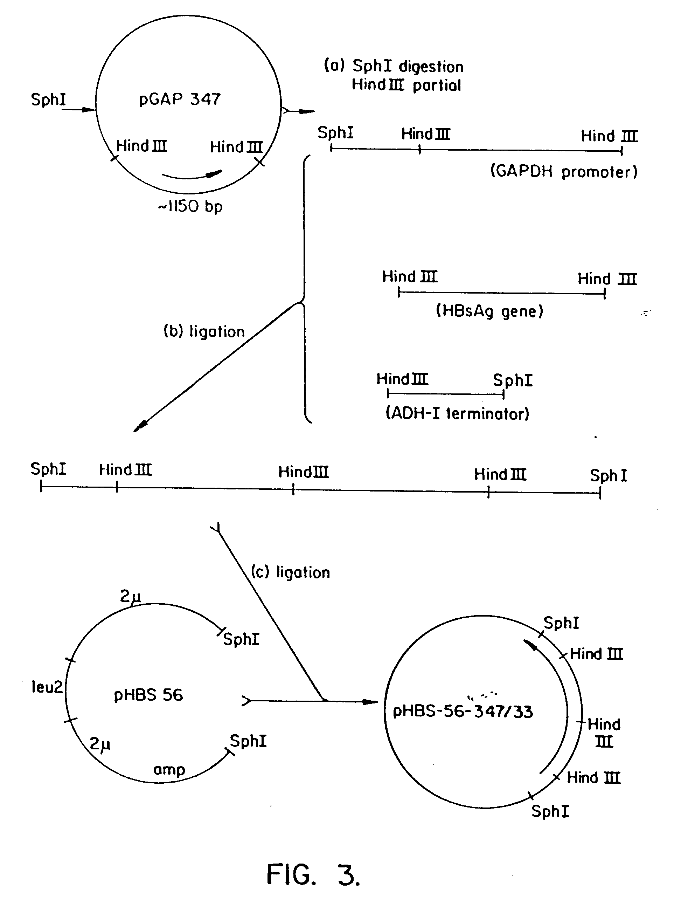

A plasmid vector (pHBS-56GAP347/33), for the expression of HBV surface antigen in yeast using the GAPDH promoter fragment was constructed as depicted in Fig. 3.

Total digestion of pGAP-347 with SphI followed by partial digestion with HindIII yielded an approximately 1700bp SphI-HindIII fragment having about 1060bp of GAPDH promoter and about 530bp of pBR322. The 1700bp SphI-HindIII GAPDH promoter fragment was ligated with the 840bp HindIII-HindIII fragment (containing the HBsAg coding region, 26 bases of 5′ non-coding region and 128bp of 3′ non-coding region, obtained from pHBS-56) and then with the 350bp HindIII-SphI fragment containing the ADH-1 termination region (isolated from pHBS-56). The 2900bp SphI fragment (cassette) was isolated and cloned in pHBS-56 previously digested with SphI. The plasmid pHBS-56 (ATCC Accession No. 40047) has been described in a co-pending application (EPA No. 82.401473.2 published as no. 72318, of Regents of the University of California) and contains the entire 2 micron plasmid, in addition to a region with the yeast leu2 gene and the amp resistance locus of pBR322. The resulting plasmid (pHBS-56GAP347/33) in which the promoter, gene and termination regions were in the proper orientations was isolated and used to transform yeast strain AB102 (MATa, pep 4-3, leu 2-3 leu2-112, ura 3-52, his 4-580, cir°) or strain 2150-2-3 (MATa, ade1, leu2-04, cir°). Strain AB102 is derived from SF657-9c by curing of 2 micron plasmids. Strain 2150-2-3 is from the collection of Dr. Leland Hartwell at the University of Washington.

Example 1

Cloning of the yeast pyruvate kinase gene.

The pyruvate kinase gene was cloned by complementation. A yeast pyruvate kinase minus mutant was transformed with a pool of recombinant YEp24 plasmids containing wild type yeast genomic DNA. The yeast strains S288C (genotype: SUC2, mal, gal2, CUP1) and pyk 1-5 (genotype: a, ade1, leu1, met14, ura3, pyk1-5) were obtained from the Yeast Genetic Stock Center, Department of Biophysics, University of California, Berkeley. The yeast genomic bank used consists of a partial Sau3A digest of total DNA from the strain S288C cloned into the BamHI site of the "shuttle" vector YEp24. The vector YEp24 contains pBR322 sequences for selection and growth in bacteria, the yeast URA3 gene for selection in yeast and an EcoRI fragment of the yeast 2μ circle to ensure plasmid replication and segregation in yeast. The pool includes sufficient independent recombinant plasmids to represent the entire yeast genome.

The strain pyk1-5 is unable to grow on medium containing glucose or lacking uracil because of mutations in this strain at the Pyk1 and URA3 loci, respectively. Transformation of this strain with the YEp24 genomic library and selection for transformants which are able to grow on medium lacking uracil and containing glucose selects for those cells which have acquired YEp24 containing the pyruvate kinase gene. Transformation of $3.5 \times 10^8$ pyk1-5 yeast cells with 10μg of YEp24 recombinant plasmid pool DNA yielded 5 independent transformants which grew in the absence of uracil and the presence of glucose.

Characterization of the insert DNA of these transformants by restriction enzyme analysis indicated that they contain overlapping DNA inserts. We focused on a single transformant, pPyK 9.1, which contained a 7.0kb insert. The pyruvate kinase gene was localized within this insert by determining which insert-specific restriction fragments hybridized to a mRNA of about 1.7kb expected for the pyruvate kinase mRNA. The localization o the PyK gene was confirmed by subcloning appropriate regions of the insert DNA and observing complementation of function in the pyk1-5 mutant. A subclone pPyK 9.1.1 which contained the Pyk gene on a 4.4kb insert was sequenced and used in expression plasmid constructions.

EXAMPLE 2

Sequence of the yeast pyruvate kinase gene.

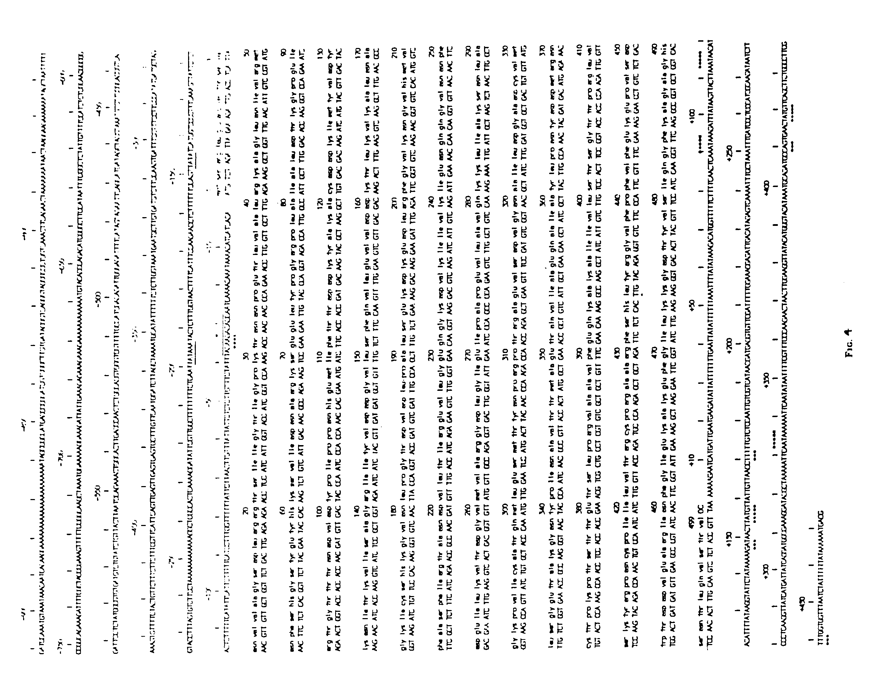

A total of 2885 nucleotides of the PyK gene have been sequenced including 1497 nucleotides in a single open reading frame, 911 nucleotides of 5′ untranslated region and 477 nucleotides of 3′ untranslated region (see Fig. 4). The gene encodes a polypeptide of 499 amino acids to give a monomer molecular weight of 54,608 daltons which agrees well with the expected value for yeast PyK. The amino acid composition derived from the nucleotide sequence also corresponds closely with that measured from the isolated yeast protein. The nucleotide sequence predicts a carboxy terminal valine which has been found for yeast pyruvate kinase.

EXAMPLE 3

Construction of yeast expression plasmids using the pyruvate kinase promoter region.

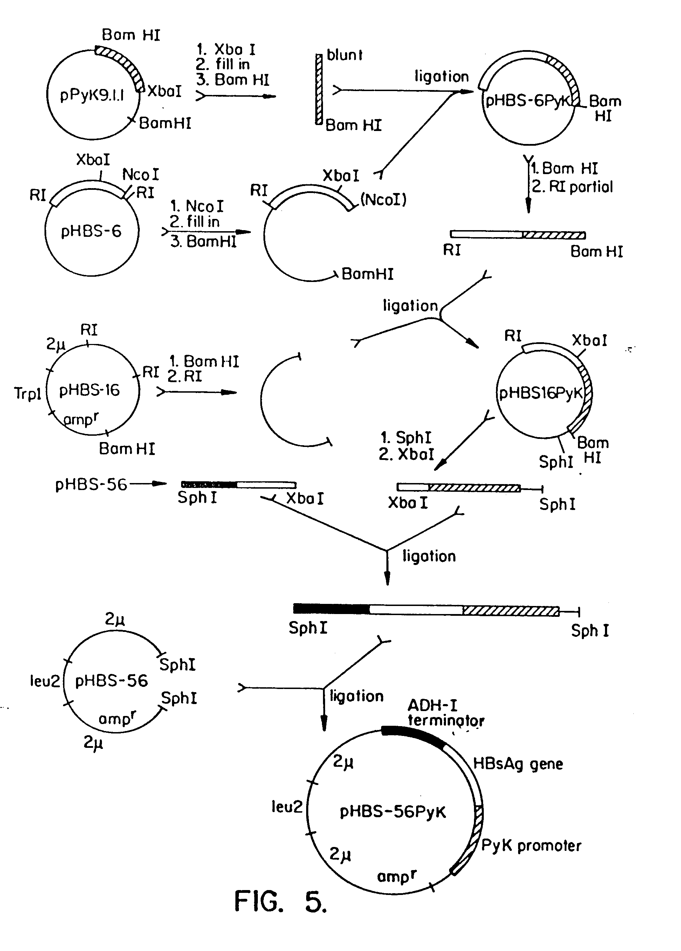

Two different constructions were made: pHBS16 PyK and pHBS56 PyK. The procedures are outlined in Fig. 5.

The plasmid pPyK 9.1.1, which contains the yeast PyK gene cloned in pBR322 was digested with XbaI and the protruding ends filled in with deoxynucleotides using DNA polymerase I. The product was digested with BamHI to finally isolate a 912bp BamHI-blunt fragment containing the PyK promoter and 8 bases from the PyK coding region. This fragment was ligated to plasmid pHBS-6 (contains the HBsAg gene, in which the 5′ non-coding region has been deleted, cloned in pBR322) previously digested with NcoI, filled in using

6

DNA polymerase and digested with BamHI. After transformation of E. coli, pHBS-6PyK was isolated. This plasmid contains the PyK promoter with codons for 3 extra amino acids fused in phase with the HBsAg coding sequence,

$$\underline{ATGTCTAG}\ \underline{CATG}\ .$$

pHBS-6PyK was digested with BamHI to completion and partially digested with EcoRI to isolate a 1750bp BamHI-EcoRI fragment containing the PyK promoter fused to the HBsAg gene. This 1750bp fragment was ligated to the large fragment obtained after digestion of pHBS-16 (ATCC Accession No. 40043, plasmid described in European Patent Application No. 82.401473.2 (EP-A-72318) with BamHI and EcoRI and used to transform E. coli. The yeast expression plasmid pHBS-16PyK was obtained. pHBS-16PyK was digested to completion with SphI and XbaI and a 1200bp SphI-XbaI fragment (containing 200bp of pBR322, the PyK promoter and 100bp of the 5′ region of the HBsAg gene) was isolated. This 1200bp SphI-XbaI fragment was ligated to a 1070bp XbaI-SphI fragment (isolated from pHBS-56) containing the 3′ end of the HBsAg gene and the ADH-1 terminator. After digestion with SphI, a SpHI-SphI 2300bp fragment (cassette) containing the PyK promoter, HBsAg gene and ADH-1 terminator was isolated. This cassette fragment was cloned in pHBS-56 which had been previously digested with SphI. The yeast expression plasmid pHBS-56 PyK was obtained. This plasmid was used to transform yeast strain AB102 (see Reference Example 2) or strain 2150-2-3 (see Reference Example 2).

EXAMPLE 4

Synthesis of HBsAg in yeast under PyK promoter control.

One hundred ml cultures of strain AB102 containing plasmid pHBS-56 PyK were grown to optical densities at 650nm of 1-2. Cell-free lysates were prepared by agitation with glass beads and removal of cell debris by centrifugation. HBsAg was measured by the Abbott Ausriall radioimmunoassay and protein concentration was determined by the Coomassie blue binding method. The results are shown in Table 2. They indicate that PyK promoter is at least two times more efficient than the ADH1 promoter for expression of protein product in yeast.

## EP 0 329 203 B1

Table 2: Synthesis of HBsAg in yeast

(a) from pHBS-56 (control, ADH-I promoter)

| Exp# | sAg (µg/ml) | protein (mg/ml) | Spec. Activity (µgsAg/mg protein) |
|------|-------------|-----------------|-----------------------------------|
| 1 | 8.2 | 24 | 0.34 |
| 2 | 7.2 | 24 | 0.32 |
| 3 | 4.7 | 27 | 0.23 |

(b) from pHBS-56 PyK (PyK promoter)

| Exp# | sAg (µg/ml) | protein (mg/ml) | Spec. Activity (µgsAg/mg protein) |
|------|-------------|-----------------|-----------------------------------|
| 1 | 18 | 2.5 | 0.68 |
| 2 | 10.6 | 22 | 0.48 |
| 3 | 15.2 | 27 | 0.56 |

Similar results were obtained by substituting yeast strain 2150-2-3 for yeast strain AB102 and repeating Example 7.

**Claims**

1. A yeast expression vector comprising a segment of foreign DNA under transcriptional control of a yeast pyruvate kinase promoter, said segment being in the correct orientation for transcription and having fewer than three codons from yeast pyruvate kinase at the 5′-end of said foreign DNA, wherein said foreign DNA codes for hepatitis B surface antigen or portion thereof.

2. A yeast expression vector of claim 1 further comprising a terminator attached to the 3′ end of the segment of foreign DNA.

3. A yeast expression vector according to claim 1, further comprising yeast two micron plasmid DNA or portion thereof.

4. The plasmid 2150-2-3-pHBS56 Pyk having ATCC accession number 20666.

5. A method of expressing a DNA coding segment in yeast, comprising the steps of:
   (a) inserting the coding segment in a yeast expression vector, said vector comprising a DNA segment derived from a yeast pyruvate kinase promoter having fewer than three codons from the 5′-end of pyruvate kinase, said promoter being adjacent to the 5′ end of the inserted DNA coding segment and so oriented that transcription initiated within said promoter includes the coding segment, thereby providing a coding segment expression vector, wherein said foreign DNA codes for hepatitis B surface antigen or portion thereof, and
   (b) transforming yeast cells with the coding segment expression vector.

6. A method according to claim 5 wherein said yeast expression vector further comprises a terminator attached to the 3′ end of the inserted DNA coding segment.

8

7. A method according to claim 6wherein said yeast expression vector further comprises a bacterial cell replication origin and is capable of replicating in a bacterial cell.

8. A method according to claim 6 wherein said terminator comprises the yeast alcohol dehydrogenase terminator.

9. A method according to claim 6 wherein said terminator comprises the yeast glyceraldehyde-3-phosphate dehydrogenase terminator.

10. A method according to claim 6 wherein said terminator comprises the yeast pyruvate kinase terminator.

## Patentansprüche

1. Hefeexpressionsvektor, enthaltend ein Segment einer Fremd-DNA unter Transkriptionskontrolle eines Hefe-Pyrovatkinasepromoters, wobei das genannte Segment sich in korrekter Orientierung zur Transkription befindet und weniger als 3 Kodons der Hefe-Pyruvatkinase am 5'-Ende der genannten Fremd-DNA aufweist und wobei die genannte Fremd-DNA für Hepatitis-B-Oberflächenantigen oder einen Teil desselben kodiert.

2. Hefeexpressionsvektor nach Anspruch 1, weiterhin enthaltend einen Terminator, welcher an das 3'-Ende des Segmentes der Fremd-DNA angeheftet ist.

3. Hefeexpressionsvektor nach Anspruch 1, weiterhin enthaltend eine Hefe-2-Mikron-Plasmid-DNA oder einen Teil derselben.

4. Plasmid 2150-2-3-pHBs56Pyk mit der ATCC-Zugriffsnummer 20666.

5. Verfahren zur Expression eines DNA-Kodierungssegmentes in Hefe mit den Schritten
   a) Insertion des Kodierungssegmentes in einen Hefeexpressionsvektor, wobei der genannte Vektor ein DNA-Segment umfaßt, das von einem Hefe-Pyruvatkinasepromoter mit weniger als 3 Kodons von 5'-Ende der Pyruvatkinase abgeleitet ist, wobei sich der Promoter benachbart zu dem 5'-Ende des insertierten DNA-Kodierungssegmentes befindet und so ausgerichtet ist, daß die innerhalb des Promoters initiierte Transkription das Kodierungssegment einschließt, wodurch ein Kodierungssegment-Expressionsvektor gebildet wird, in dem die Fremd-DNA für Hepatitis-B-Oberflächenantigen oder einen Teil desselben kodiert, und
   b) Transformation von Hefezellen mit dem Kodierungssegment-Expressionsvektor.

6. Verfahren nach Anspruch 5, wobei der genannte Hefeexpressionsvektor weiterhin einen an das 3'-Ende des insertierten DNA-Kodierungssegmentes angehefteten Terminator umfaßt.

7. Verfahren nach Anspruch 6, wobei der genannte Hefeexpressionsvektor weiterhin einen Bakterienzellen-Replikationsorigin aufweist und zur Replikation in einer Bakterienzelle befähigt ist.

8. Verfahren nach Anspruch 6, wobei der Terminator den Hefe-Alkoholdehydrogenase-Terminator umfaßt.

9. Verfahren nach Anspruch 6, wobei der Terminator den Hefe-Glyceraldehyd-3-phosphatdehydrogenase-Terminator umfaßt.

10. Verfahren nach Anspruch 6, wobei der Terminator den Pyruvatkinase-Terminator umfaßt.

## Revendications

1. Vecteur d'expression de levure comprenant un segment d'ADN étranger sous le contrôle transcriptionnel d'un promoteur pyruvate kinase de levure, ce segment étant dans l'orientation correcte pour la transcription et ayant moins de trois codons provenant de pyruvate kinase de levure à l'extrémité 5' de cet ADN étranger, dans lequel cet ADN étranger code pour l'antigène de surface de l'hépatite B ou une portion de celui-ci.

**2.** Vecteur d'expression de levure suivant la revendication 1, comprenant de plus un élément de terminaison fixé à l'extrémité 3' du segment de l'ADN étranger.

**3.** Vecteur d'expression de levure suivant la revendication 1, comprenant de plus un ADN de plasmide 2 micron de levure ou une portion de celui-ci.

**4.** Plasmide 2150-2-3-pHB556 Pyk ayant le numéro d'accès ATCC 20666.

**5.** Méthode pour l'expression d'un segment codant pour un ADN dans une levure, comprenant les étapes suivantes :

(a) insertion du segment codant dans un vecteur d'expression de levure, ce vecteur comprenant un segment d'AN dérivé d'un promoteur pyruvate kinase de levure ayant moins de trois codons provenant de l'extrémité 5' de pyruvate kinase, ce promoteur étant adjacent à l'extrémité 5' du segment inséré codant pour un ADN et orienté de telle sorte qu'une transcription initiée à l'intérieur de ce promoteur comprend le segment codant, de façon à fournir un vecteur d'expression du segment codant, dans lequel cet ADN étranger code pour l'antigène de surface de l'hépatite B ou une portion de celui-ci, et

(b) transformation des cellules de levure avec le vecteur d'expression du segment codant.

**6.** Méthode suivant la revendication 5, dans laquelle ce vecteur d'expression de levure comprend de plus un élément de terminaison attaché à l'extrémité 3' du segment inséré codant pour un ADN.

**7.** Méthode suivant la revendication 6, dans laquelle ce vecteur d'expression de levure comprend de plus une origine de réplication de cellule bactérienne et est capable de se répliquer dans une cellule bactérienne.

**8.** Méthode suivant la revendication 6, dans laquelle cet élément de terminaison comprend l'élément de terminaison alcool déshydrogénase de levure.

**9.** Méthode suivant la revendication 6, dans laquelle cet élément de terminaison comprend l'élément de terminaison I-glycéraldéhyde-3-phosphate déshydrogénase de levure.

**10.** Méthode suivant la revendication 6, dans laquelle cet élément de terminaison comprend l'élément de terminaison pyruvate kinase de levure.

FIG. 1.

DNA 347

```
        10         20         30         40         50         60
AAGCTTACCA GTTCTCACAC GGAACACCAC TAATGGACAC AAATTCGAAA TACTTTGACC

        70         80         90        100        110        120
CTATTTTCGA GGACCTTGTC ACCTTGAGCC CAAGAGAGCC AAGATTTAAA TTTTCCTATG

       130        140        150        160        170        180
ACTTGATGCA AATTCCCAAA GCTAATAACA TGCAAGACAC GTACGGTCAA GAAGACATAT

       190        200        210        220        230        240
TTGACCTCTT AACTGGTTCA GACGCGACTG CCTCATCAGT AAGACCCGTT GAAAAGAACT

       250        260        270        280        290        300
TACCTGAAAA AAACGAATAT ATACTAGCGT TGAATGTTAG CGTCAACAAC AAGAAGTTTA

       310        320        330        340        350        360
ATGACGCGGA GGCCAAGGCA AAAAGATTCC TTGATTACGT AAGGGAGTTA GAATCATTTT

       370        380        390        400        410        420
GAATAAAAAA CACGCTTTTT CAGTTCGAGT TTATCATTAT CAATACTGCC ATTTCAAAGA

       430        440        450        460        470        480
ATACGTAAAT AATTAATAGT AGTGATTTTC CTAACTTTAT TTAGTCAAAA ATTAGCCTTT

       490        500        510        520        530        540
TAATTCTGCT GTAACCCGTA CATGCCCAAA ATAGGGGGCG GGTTACACAG AATATATAAC

       550        560        570        580        590        600
ATCGTAGGTG TCTGGGTGAA CAGTTTATCC CTGGCATCCA CTAAATATAA TGGAGCTCGC

       610        620        630        640        650        660
TTTTAAGCTG GCATCCAGAA AAAAAAGAA TCCCAGCACC AAAATATTGT TTTCTTCACC

       670        680        690        700        710        720
AACCATCAGT TCATAGGTCC ATTCTCTTAG CGCAACTACA GAGAACAGGG GCACAAACAG

       730        740        750        760        770        780
GCAAAAACG GGCACAACCT CAATGGAGTG ATGCAACCTG CCTGGAGTAA ATGATGACAC

       790        800        810        820        830        840
AAGGCAATTG ACCCACGCAT GTATCTATCT CATTTTCTTA CACCTTCTAT TACCTTCTGC

       850        860        870        880        890        900
TCTCTCTGAT TTCGAAAAAG CTGAAAAAAA AGGTTGAAAC CAGTTCCCTG AAATTATTCC

       910        920        930        940        950        960
CCTACTTGAC TAATAAGTAT ATAAAGACGG TAGGTATTGA TTGTAATTCT GTAAATCTAT

       970        980        990       1000       1010       1020
TTCTTAAACT TCTTAAATTC TACTTTTATA GTTAGTCTTT TTTTAGTTT TAAAACACCA

      1030       1040       1050       1060
AGAACTTAGT TTCGAATAAA CACACATAAA CAAACAAGCT T
```

FIG. 2

12

FIG. 3.

Fig. 4

FIG. 5.